Europäisches Patentamt

European Patent Office

Office européen des brevets

(11) Publication number: **0 337 162**
**A1**

## (12) EUROPEAN PATENT APPLICATION

(21) Application number: **89104931.4**

(22) Date of filing: **20.03.89**

(51) Int. Cl.⁴: **A61B 5/02**

(30) Priority: **24.03.88 US 172557**

(43) Date of publication of application:
**18.10.89 Bulletin 89/42**

(84) Designated Contracting States:
**CH DE FR GB IT LI**

(71) Applicant: **SpaceLabs, Inc.**
**4200 - 150th Avenue N.E.**
**Redmond Washington 97013(US)**

(72) Inventor: **Sorensen, Jay Randall**
**17941 S.W. Sandra Lane**
**Aloha Oregon 97006(US)**
Inventor: **Millay, Jack McDonald**
**Route 3, Box 1143**
**Beaverton Oregon 97007(US)**

(74) Representative: **Patentanwälte Grünecker,**
**Kinkeldey, Stockmair & Partner**
**Maximilianstrasse 58**
**D-8000 München 22(DE)**

(54) **Anti-collapsing tube for blood pressure cuffs.**

(57) An anti-collapsing tube for connecting a blood pressure cuff to a blood pressure measuring device (11). One embodiment of the anti-collapsing tube has at least one longtudinally extending ridge (26), and preferably a plurality of circumferentially spaced, longitudinally extending ridges formed on its inner surface (16). The ridges prevent opposite, abutting inner surfaces of the tube from contacting each other over their entire surfaces to block the tube. A second embodiment of the anti-collapsing tube has a string (56) extending through its lumen to space apart at least a portion of opposite inner surfaces of the tube at the bend or kink so that opposite, abutting inner surfaces of the tubing cannot contact each other over their entire surfaces to block the lumen.

FIG.1

## ANTI-COLLAPSING TUBE FOR BLOOD PRESSURE CUFFS

Field of the Invention

This invention relates to blood pressure monitoring, and more particularly, to a tube for use with a blood presure cuff that is incapable of collapsing shut if the tube becomes bent or kinked.

Background Art

Blood pressure is normally measured by placing a blood pressure cuff around the arm of a patient over the brachial artery. The cuff typically includes an inflatable bladder. The bladder is inflated through a tube extending from the bladder to compress the arm of the patient, thereby pinching off the flow of blood through the brachial artery. The pressure in the bladder is gradually reduced while measuring the pressure and listening for sounds caused by the flow of blood through the brachial artery. When blood flow is detected during systole, the air pressure in the bladder is recorded as the systolic blood pressure. Similarly, when blood flow is detected during diastole, the air pressure in the bladder is recorded as the diastolic blood pressure.

The most common device for measuring blood pressure using the above-described procedure is the familiar manually pumped cuff which uses a mercury manometer as the pressure measuring device. After the cuff is inflated with air, the pressure in the cuff is gradually reduced, while a stethoscope is used to detect the flow of blood in the brachial artery beneath the cuff.

Automated patient monitoring systems are also in common use to allow blood pressure measurements to be periodically taken without the assistance of medical personnel. In these automated systems, a blood pressure cuff is periodically inflated and the cuff pressure is then gradually reduced while the blood pressure is measured. An electrical or acoustic audio pickup device listens for the sound generated as blood starts flowing through the brachial artery to determine the systolic and diastolic blood pressures.

The automated blood pressure monitors described above are frequently worn by patients throughout the day and night, including while the patient is sleeping. Patients will not generally wake up each time that the blood pressure cuff is inflated through the tube extending to the cuff from the monitor. However, inflation of the cuff can sometimes disturb a patient's sleep to the extent that he or she will roll over or otherwise change his or her sleeping position. Under these circumstances, it is possible to bend or kink the tube extending to the pressure cuff after the cuff has been inflated. The tubes typically used with blood pressure cuff bladders are pliable and, therefore, can kink or bend. This bend or kink in the tube can cause the tube to collapse, thereby totally blocking the tube. Blocking the tube not only interferes with the measurement being taken, but it can prevent the air from escaping from the cuff so that the cuff remains inflated for a prolonged period. The pressure of the cuff can then cut off all blood flow through the brachial artery until either the air has finally leaked through the blockage or the patient wakes up and unbends or unkinks the tube. However, the prolonged blockage of the brachial artery can cause patient discomfort until the patient's circulation returns to normal.

Although the problem of blood pressure tube collapse is most common where the patient collapses the tube by shifting position after the tube has become inflated, it can also occur if the tube is bent or kinked before inflation if the air pump in the monitor is sufficiently powerful to force air past the bend or kink.

Disclosure of the Invention

It is an object of the invention to provide a tube for a blood pressure cuff that cannot be blocked if the tube becomes inadvertently bent or kinked.

It is another object of the invention to provide an anti-collapsing tube of a blood pressure cuff that is inexpensive and sufficiently sturdy that the cuff and tube can withstand heavy use.

These and other objects of the invention are provided by an anti-collapsing tube for use with a blood pressure cuff. In one embodiment, the tube includes a length of cylindrical tubing having a non-smooth inner surface surrounding a lumen of the tubing. In the event that the tubing kinks or bends, the opposite abutting inner surfaces of the tubing cannot contact each other over their entire surfaces to block the lumen. Although the inner surface of the tubing may have a variety of configurations, it preferably includes at least one longitudinal ridge and preferably a plurality of circumferentially spaced, longitudinal ridges.

In another embodiment, the tube may have a smooth inner surface surrounding a lumen, and a spacer member, such as a string, extends through the lumen. The spacing member has a cross-sectional area that is substantially smaller than the cross-sectional area of the lumen so that the spacing member does not substantially interfere with airflow through the tubing. However, in the event that the tubing kinks or bends, the spacing member spaces apart at least a portion of opposite inner surfaces of the tubing at the bend or kink so that opposite abutting inner surfaces of the tubing cannot contact each other over their entire surfaces to block the lumen.

Brief Description of the Drawings

Figure 1 is a plan view of a blood pressure cuff bladder utilizing one embodiment of the inventive anti-collapsing tube extending from the bladder.

Figure 2 is a cross-sectional view taken along the line 22 of Figure 1 showing the tube in its normal condition.

Figure 3 is a cross-sectional view taken along the line 33 of Figure 1 showing the tube in its collapsed condition resulting from a bend in the tube.

Figure 4 is a plan view of a blood pressure cuff bladder utilizing another embodiment of the inventive anti-collapsing tube extending from the bladder.

Figure 5 is a cross-sectional view taken along the line 55 of Figure 4 showing the tube in its normal condition.

Figure 6 is a cross-sectional view taken along the line 66 of Figure 4 showing the tube in its collapsed condition resulting from a bend in the tube.

Best Mode for Carrying Out the Invention

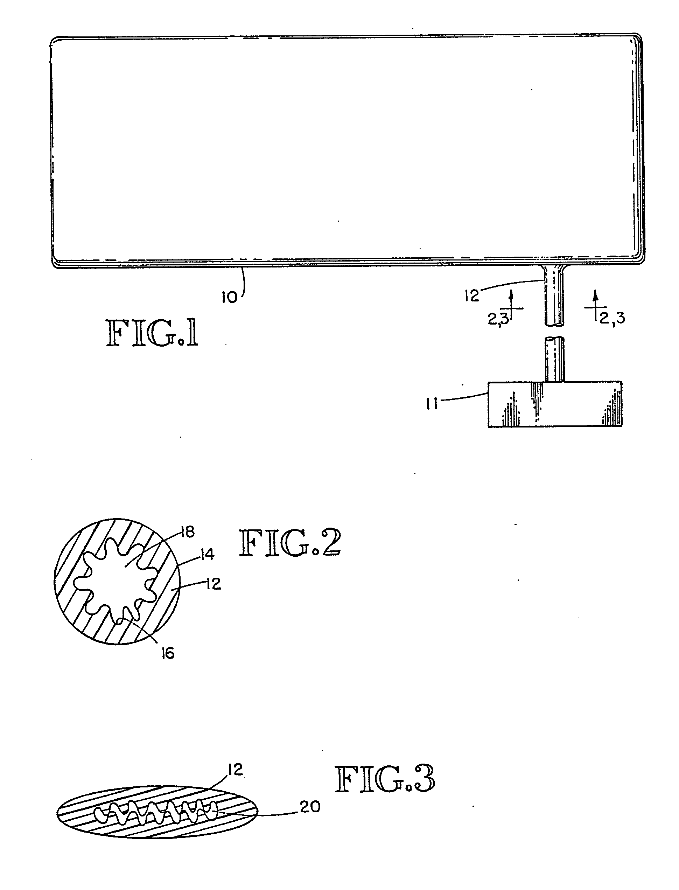

One embodiment of an anti-collapsing tube extending to the bladder of a blood pressure cuff is illustrated in Figure 1. The cuff includes a resilient, relatively thin bladder 10 having a generally rectangular shape. As is well known in the art, the bladder 10 is placed in a casing (not shown), and the casing is then wrapped snugly around the arm of the patient. The bladder 10 is connected to a blood pressure measuring device 11 through a pliable tube 12. The blood pressure measuring device may be of either the manual or the automatic type, but the advantages of the inventive tube will be most beneficial when the tube 12 is used with an automatic blood pressure measuring device 11. When a measurement is to be made, the measuring device 11 inflates the bladder 10 through the tube 12, thereby cutting off the flow of blood through the brachial artery. If the tube 12 becomes bent or kinked after the bladder has been inflated, it can collapse, thereby blocking the tube 12 and preventing the air from being vented from the bladder 10.

The tubes typically used with blood pressure cuffs normally have smooth inner and outer walls. Therefore, when the tube becomes bent or kinked, the opposite inner walls can flushly contact each other, thereby totally blocking the tube. As best illustrated in Figure 2, the inventive tube 12 for use with blood pressure cuffs has an outer surface 14 and an inner surface 16 surrounding a lumen 18. A plurality of longitudinal ridges 20 are formed on the inner surface 16. As a result, when the tube 12 becomes bent, as illustrated in Figure 3, the ridges 20 form air passages therebetween so that air can flow through the tube 12 even though it is bent or kinked. Although the tube 12 illustrated in Figures 1-3 has an inner surface 16 containing regularly spaced longitudinal ridges 20, it will be understood that any non-smooth wall configuration can be used as long as the wall configuration forms an air passage if the tube 12 becomes bent or kinked.

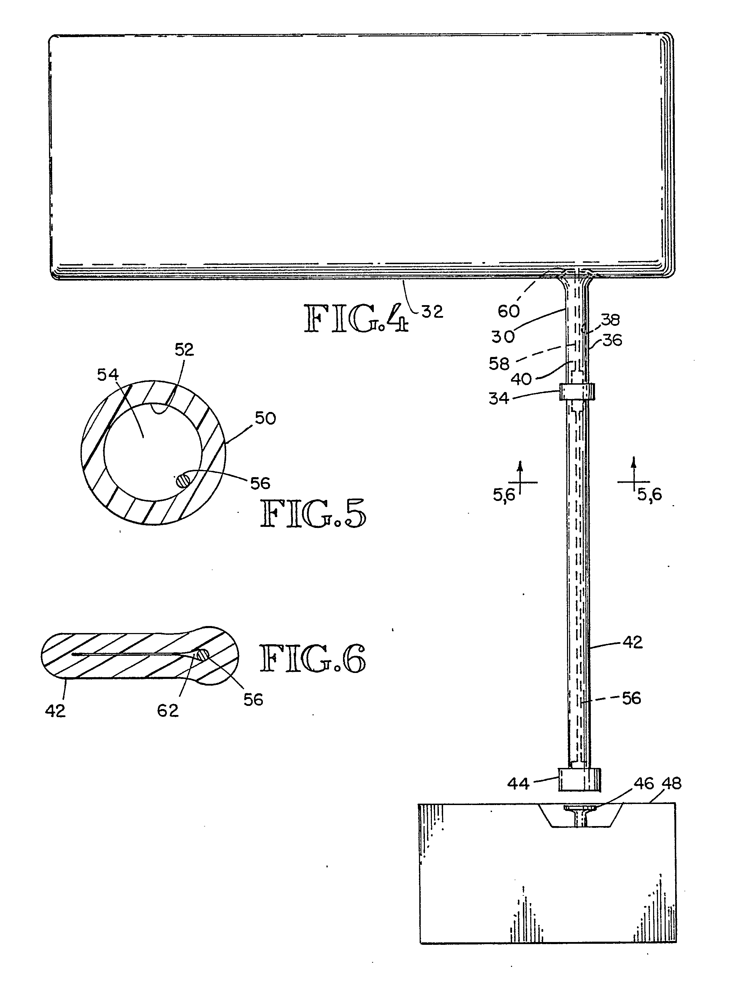

Another embodiment of an anti-collapsing tube for use with a blood pressure cuff is illustrated in Figures 4 and 5. In this embodiment, a relatively short tube 30 extends from a conventional blood pressure cuff bladder 32 to a conventional blood pressure cuff coupling 34. The tube 30 is cylindrical in shape, and it has a smooth outer surface 36 and a smooth inner surface 38 surrounding a lumen 40. A second, substantially longer tube 42 extends from the coupling 34 to another coupling 44 that attaches to a conventional fitting 46 on a blood pressure monitor 48. The tube 42, like the short tube 30, is cylindrical in shape, and it has a smooth outer surface 50 and a smooth inner surface 52 surrounding a lumen 54.

A string 56 or similar member extends through the tube 42 with its ends attached to the couplings 34, 44. Similarly, a string 58 or similar member extends through the tube 30 from the coupling 34 to a support member 60 formed in the bladder 32. The strings 56, 58 may be fabricated from any suitable material, and it may be solid, tubular or braided.

Referring to Figure 6, the string 56 prevents the lumen 54 of the tube 42 from totally collapsing in the event that the tube 42 becomes bent or kinked as illustrated in Figure 6. Instead, the string 56 spaces the opposed inner surfaces 52 walls of the tube 42 from each other to form an air passage 62. The air passage 62 thus allows the bladder 32 to vent if the tube 42 becomes bent or kinked.

Claims

1. An anti-collapsing tube for use with a blood pressure cuff, said tube comprising a length of cylindrical tubing having an outer surface and an inner surface surrounding a lumen of said tubing, said tube further including an elongated spacing member extending through said lumen and being substantially unconnected to said tubing between its ends, said spacing member having a cross-sectional area that is substantially smaller than the cross-sectional area of said lumen, whereby said spacing member does not substantially interfere with airflow through said tubing, yet, in the event that said tubing kinks or bends, said spacing member spaces apart at least a portion of opposite inner surfaces at said bend or kink so that opposite, abutting inner surfaces of said tubing cannot contact each other over their entire surfaces to block said lumen.

2. The anti-collapsing tube of claim 1 wherein said spacing member comprises a length of string.

3. The anti-collapsing tube of claim 1 wherein said tube further includes a pair of couplings at opposite ends and wherein the ends of said spacing member are secured to respective couplings of said tubing.

4. In a blood pressure cuff having a bladder with a pneumatic port, a blood pressure measuring device having a pneumatic port and a relatively pliant blood pressure tube extending between the pneumatic port of said bladder and the pneumatic port of said blood pressure measuring device, said tube having an outer surface and an inner surface surrounding a lumen through which air is adapted to flow, a method of preventing said lumen from becoming blocked by a bend or a kink in said tube, said method comprising placing an elongated spacing member through substantially the entire length of said lumen, said spacing member being substantially unconnected to said tubing between its ends, said spacing member having a cross-sectional area that is substantially smaller than the cross-sectional area of said lumen, whereby said spacing member does not substantially interfere with airflow through said tubing, yet, in the event that said tubing kinks or bends, said spacing member spaces apart at least a portion of opposite inner surfaces at said bend or kink so that opposite, abutting inner surfaces of said tubing cannot contact each other over their entire surfaces to block said lumen.

5. The method of claim 4 wherein said spacing member comprises a length of string.

6. The method of claim 4 wherein said tube further includes a pair of couplings at opposite ends and wherein the ends of said spacing member are secured to respective couplings of said tubing.

FIG.1

FIG.2

FIG.3

FIG.4

FIG.5

FIG.6

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| A | DE-A-2 340 611 (UNITED STATES SURGICAL CORP.) * page 6, line 19 - page 9, line 21; figures 1-4 * | 1,4 | A 61 B 5/02 |

TECHNICAL FIELDS SEARCHED (Int. Cl.4)

A 61 B 5/00
A 61 B 7/00
A 61 M 1/00
A 61 M 25/00
A 62 B 9/00

The present search report has been drawn up for all claims

| Place of search | Date of completion of the search | Examiner |
|---|---|---|
| BERLIN | 15-06-1989 | WEIHS J.A. |

EPO FORM 1503 03.82 (P0401)